(19) Europäisches Patentamt
European Patent Office
Office européen des brevets

(11) EP 1 749 525 A1

(12) **EUROPEAN PATENT APPLICATION**

(43) Date of publication:
**07.02.2007 Bulletin 2007/06**

(21) Application number: **05384009.6**

(22) Date of filing: **15.07.2005**

(51) Int Cl.:
***A61K 31/415*** *(2006.01)* ***A61K 31/4152*** *(2006.01)*
***A61P 25/30*** *(2006.01)* ***A61P 25/32*** *(2006.01)*
***A61P 25/34*** *(2006.01)* ***A61P 25/36*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **LABORATORIOS DEL DR. ESTEVE, S.A.**
**08041 Barcelona (ES)**

(72) Inventor: **Buschmann, Helmut Heinrich**
**08960 Sant Just Desvern (Barcelona) (ES)**

(54) **Combination of substituted pyrazolines and anti-addictive agent**

(57) The present invention relates to an active substance combination comprising at least one substituted pyrazoline compound and at least one anti-addictive compound, a medicament comprising said active substance combination, a pharmaceutical formulation comprising said active substance combination and the use of said active substance combination for the manufacture of a medicament.

EP 1 749 525 A1

## Description

The present invention relates to an active substance combination comprising at least one substituted pyrazoline compound and at least one anti-addictive agent, a medicament comprising said active substance combination, a pharmaceutical formulation comprising said active substance combination and the use of said active substance combination for the manufacture of a medicament.

Addiction, particularly addiction to nicotine, alcohol or drugs, is widespread and represents one of the most pressing health problems among the populations of the developed world. Thus, there is still a large demand for medicaments that are suitable for preventing or ameliorating addiction.

Thus, it was an object of the present invention to provide a medicament that is suitable for the prophylaxis and/or treatment of addiction.

It has now surprisingly been found that the pharmacological efficacy of an anti-addictive agent is supported by their administration in combination with one or more substituted pyrazoline compounds of general formula I and/or I' given below.

Thus, in one of its aspects the present invention relates to an active substance combination comprising

(A) at least one substituted pyrazoline compound of general formula I

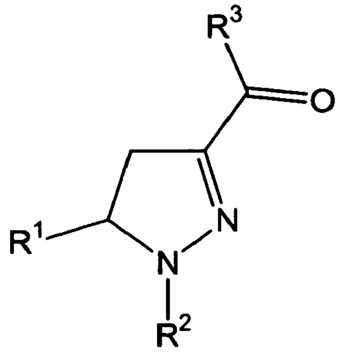

I

wherein

$R^1$ represents an optionally at least mono-substituted phenyl group;

$R^2$ represents an optionally at least mono-substituted phenyl group;

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an —$NR^4R^5$-moiety,

$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group; an —$SO_2$-$R^6$-moiety; or an -$NR^7R^8$-moiety, with the proviso that $R^4$ and $R^5$ do not identically represent hydrogen;

$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono- or polycyclic ring system and/or bonded via a linear or branched alkylene group;

$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with

an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
and/or
at least one substituted pyrazoline compound of general formula I',

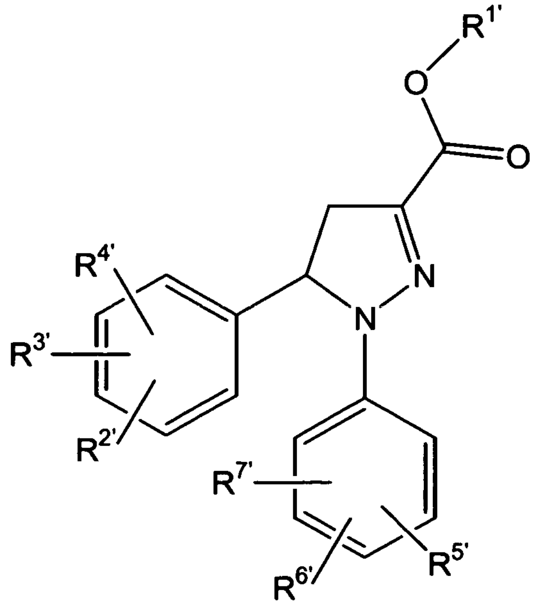

I'

wherein
$R^{1'}$ represents hydrogen or a linear or branched $C_{1-4}$-alkyl group,
$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{8'}$, SH, $SR^{8'}$, $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched $C_{1-6}$ alkyl,
$R^{5'}$ and $R^{6'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, - (C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
$R^{7'}$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
and

(B) at least one anti-addictive agent.

Preferably the active substance combination according to the present invention comprises one or more substituted pyrazoline compounds of general formula I given above, wherein $R^1$ represents a phenyl group, which is optionally

substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R" whereby R' and R" for each substituent independently represent linear or branched $C_{1-6}$ alkyl, preferably $R^1$ represents a phenyl group, which is optionally substituted by one or more substituents selected from the group consisting of methyl, ethyl, F, Cl, Br and $CF_3$, more preferably $R^1$ represents a phenyl group, which is mono-substituted with a chlorine atom in the 4-position.

Also, preferably the active substance combination according to the present invention comprises one or more substituted pyrazoline compounds of general formula I given above, wherein $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and optionally R" for each substituent independently represent linear or branched $C_{1-6}$ alkyl, preferably $R^2$ represents a phenyl group, which is optionally substituted by one or more substituents independently selected from the group consisting of methyl, ethyl, F, Cl, Br and $CF_3$, more preferably $R^2$ represents a phenyl group, which is di-substituted with two chlorine atoms in its 2- and 4-position.

Also, preferably the active substance combination according to the present invention comprises one or more substituted pyrazoline compounds of general formula I given above, wherein $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be system, or $R^3$ represents an —$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an -$NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an -$NR^4R^5$-moiety.

Also, preferably the active substance combination according to the present invention comprises one or more substituted pyrazoline compounds of general formula I given above, wherein $R^4$ and $R^5$, identical or different, represent a hydrogen atom; an unbranched or branched, saturated or unsaturated, optionally at least mono-substituted $C_{1-6}$-aliphatic radical; a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system and/or bonded via a methylene (-$CH_2$-) or ethylene (-$CH_2$-$CH_2$)-group; an -$SO_2$-$R^6$-moiety; or an -$NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system; an -$SO_2$-$R^6$-moiety; or an -$NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an -$SO_2$-$R^6$-moiety; or an -$NR^7R^8$-moiety, or $R^4$ and $R^5$, identical or different, represent a methyl group, an ethyl group, an n-propyl group, an isopropyl group, an n-butyl group, a sec-butyl group or a tert.-butyl group.

Also, preferably the active substance combination according to the present invention comprises one or more substituted pyrazoline compounds of general formula I given above, wherein $R^6$ represents a linear or branched, saturated or unsaturated, optionally at least mono-substituted $C_{1-6}$ aliphatic group; a saturated or unsaturated, optionally at least mono-substituted, optionally at least one heteroatom as ring member containing $C_{3-8}$ cycloaliphatic group, which may be condensed with a mono- or polycyclic ring-system; or an optionally at least mono-substituted, 5- or 6-membered aryl or heteroaryl group, which may be condensed with a mono- or polycyclic ring system and/or bonded via a methylene (-$CH_2$-) or ethylene (-$CH_2$-$CH_2$)-group, preferably $R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups.

Also, preferably the active substance combination according to the present invention comprises one or more substituted pyrazoline compounds of general formula I given above, wherein $R^7$ and $R^8$, identical or different, represent a hydrogen atom; an unbranched or branched, saturated or unsaturated, optionally at least mono-substituted $C_{1-6}$ aliphatic radical; a saturated or unsaturated, optionally at least mono-substituted, optionally at least one heteroatom as ring member containing $C_{3-8}$ cycloaliphatic group, which may be condensed with an optionally at least mono-substituted

mono- or polycyclic ring system; or an optionally at least mono-substituted, 5- or 6 membered aryl or heteroaryl group, which may be condensed with an optionally at least mono-substituted mono- or polycyclic ring system and/or bonded via a methylene ($-CH_2-$) or ethylene ($-CH_2-CH_2$)-group, preferably $R^7$ and $R^8$, identical or different, represent a hydrogen atom or a $C_{1-6}$ alkyl radical.

More preferably the active substance combination according to the present invention comprises one or more substituted pyrazoline compounds of general formula I given above, wherein
$R^1$ represents a phenyl group, which is optionally substituted with 1, 2, 3, 4 or 5 substituents independently selected from the group consisting of methyl, ethyl, F, Cl, Br and $CF_3$,
$R^2$ represents a phenyl group, which is optionally substituted with 1, 2, 3, 4 or 5 substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more of $C_{1-6}$-alkyl groups, or $R^3$ represents an $-NR^4R^5$-moiety,
$R^4$ represents a hydrogen atom or a linear or branched $C_{1-6}$-alkyl group,
$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different.
Also, more preferably the active substance combination according to the present invention comprises one or more substituted pyrazoline compounds of general formula I given above, wherein
$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group, a piperidinyl group, a piperazinyl group, a homo-piperazinyl group, a morpholinyl group, or an $-NR^4R^5$-moiety,
$R^4$ represents a hydrogen atom or a linear or branched $C_{1-6}$-alkyl group,
$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different.
Yet more preferably, the active substance combination according to the present invention comprises one or more substituted pyrazoline compounds of general formula I given above selected from the group consisting of
N-piperidinyl-5-(4-chloro-phenyl)-1-(2,4-dichlorophenyl)-4,5-dihydro-1 H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1 H-pyrazole-3-carboxylic acid-(4-methyl-piperazin-1-yl)-amide,
5-(4-Chloro-phenyl)-1-(2,4-dichloro-phenyl)-4,5-dihydro-1 H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone and
N-[5-(4-Chloro-phenyl)-1-(2,4-dichlorophenyl)-4,5-dihydro-1H-pyrazole-3-carbonyl]-4-methylphenylsulfonamide,
optionally in form of one of the stereoisomers, preferably enantiomers or stereoisomers, preferably enantiomers and/or diastereomers, in any mixing ratio, or a corresponding N-oxide thereof, or a corresponding salt thereof, or a corresponding solvate thereof.

Also, more preferably the active substance combination according to the present invention may as the pyrazoline compound of general formula I comprise one of the following compounds

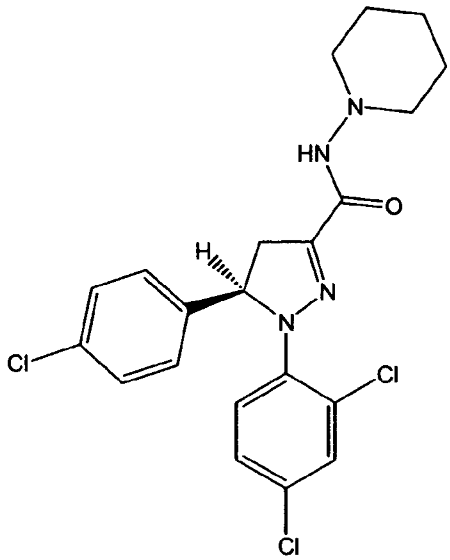 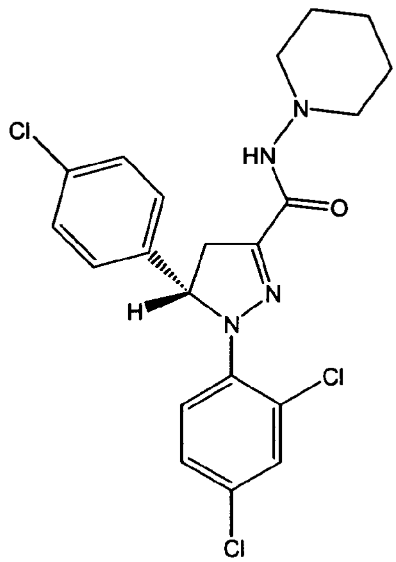

in each case optionally in the form of a corresponding N-oxide, a corresponding salt or a corresponding solvate thereof.

Also, preferably the active substance combination according to the present invention comprises one or more substituted pyrazoline compounds of general formula I' given above, wherein at least one of $R^{2'}$, $R^{3'}$ or $R^{4'}$ represents hydrogen, while at least one of $R^{2'}$, $R^{3'}$ or $R^{4'}$ is different from hydrogen.

Also, preferably the active substance combination according to the present invention comprises one or more substituted pyrazoline compounds of general formula I' given above, wherein $R^{7'}$ represents hydrogen.

Also, preferably the active substance combination according to the present invention comprises one or more substituted pyrazoline compounds of general formula I' given above, wherein $R^{2'}$, $R^{3'}$ and $R^{4'}$ independently of each other represent hydrogen, a linear or branched $C_{1-6}$-alkyl group, a halogen atom, or $CF_3$, preferably $R^{2'}$, $R^{3'}$ and $R^{4'}$ independently of each other represent hydrogen, methyl, ethyl, F, Cl, Br and $CF_3$.

Also, preferably the active substance combination according to the present invention comprises one or more substituted pyrazoline compounds of general formula I' given above, wherein $R^{5'}$ and $R^{6'}$ independently of each other represent a linear or branched $C_{1-6}$-alkyl group, a halogen atom, or $CF_3$, preferably $R^{5'}$ and $R^{6'}$ independently of each other represent methyl, ethyl, F, Cl, Br and $CF_3$.

Also, preferably the active substance combination according to the present invention comprises one or more substituted pyrazoline compounds of general formula I' given above, wherein $R^{2'}$ represents a chlorine atom in the 4-position of the phenyl ring, while $R^{3'}$ and $R^{4'}$ represent hydrogen.

Also, preferably the active substance combination according to the present invention comprises one or more substituted pyrazoline compounds of general formula I' given above, wherein $R^{5'}$ and $R^{6'}$ each represent a chlorine atoms in the 2- and 4-position of the phenyl ring, while $R^{7'}$ represents hydrogen.

Also, preferably the active substance combination according to the present invention comprises one or more substituted pyrazoline compounds of general formula I' given above, wherein $R^{1'}$ represents hydrogen, methyl or ethyl, preferably hydrogen.

Also, preferably the active substance combination according to the present invention comprises one or more substituted pyrazoline compounds of general formula I' given above, wherein the compounds of general formula I' are represented by the following structure II:

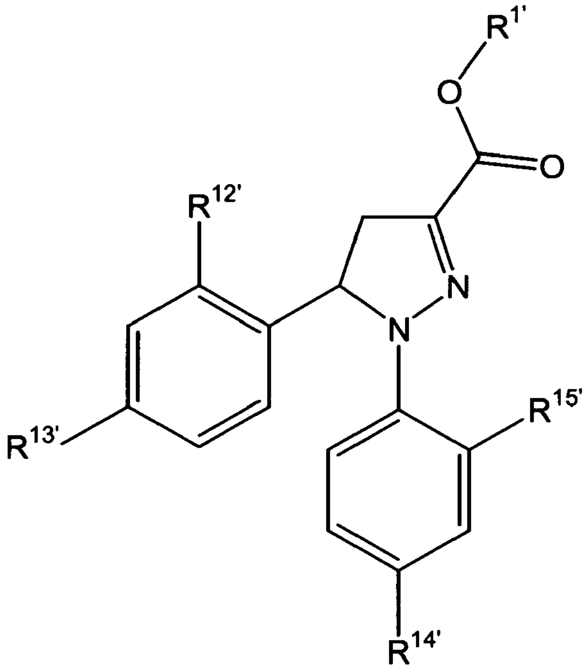

II

wherein
$R^{1'}$ represents hydrogen or a linear or branched $C_{1-4}$-alkyl group,
$R^{12'}$ or $R^{13'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,
$R^{14'}$ or $R^{15'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also, preferably the active substance combination according to the present invention comprises one or more substituted pyrazoline compounds of general formula I' given above, wherein in general structure II $R^{12'}$ and $R^{13'}$ independently of each other represent hydrogen, a linear or branched $C_{1-6}$-alkyl group, a halogen atom, or $CF_3$, preferably $R^{12'}$ and $R^{13'}$ independently of each other represent hydrogen, methyl, ethyl, F, Cl, Br and $CF_3$.

Also, preferably the active substance combination according to the present invention comprises one or more substituted pyrazoline compounds of general formula I' given above, wherein in general structure II $R^{14'}$ and $R^{15'}$ independently of each other represent a linear or branched $C_{1-6}$-alkyl group, a halogen atom, or $CF_3$, preferably $R^{14'}$ and $R^{15'}$ independently of each other represent methyl, ethyl, F, Cl, Br and $CF_3$.

Also, preferably the active substance combination according to the present invention comprises one or more substituted pyrazoline compounds of general formula I' given above, wherein in general structure II $R^{13'}$ represents Cl and $R^{12'}$ represents hydrogen.

Also, preferably the active substance combination according to the present invention comprises one or more substituted pyrazoline compounds of general formula I' given above, wherein in general structure II $R^{14'}$ and $R^{15'}$ each represent Cl.

Also, preferably the active substance combination according to the present invention comprises one or more substituted pyrazoline compounds of general formula I' given above, wherein in general structure II $R^{1'}$ represents hydrogen, methyl or ethyl, preferably hydrogen.

Also, preferably the active substance combination according to the present invention comprises the compound 5-(4-chloro-phenyl)-1-(2,4-dichlorophenyl)-4,5-dihydro-1 H-pyrazol-3-carboxylic acid,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof as compound of general formula I'.

Also, more preferably the active substance combination according to the present invention may as the pyrazoline

compound of general formula I' comprise one of the following compounds

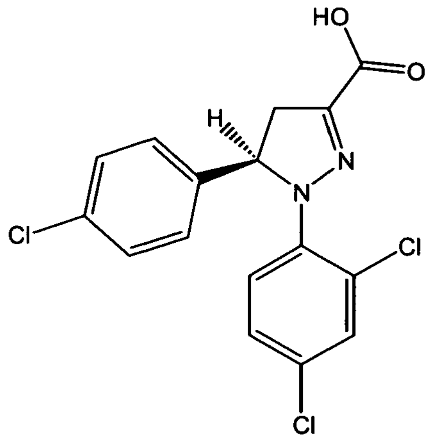

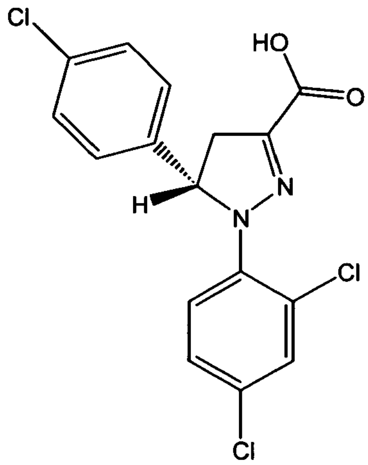

in each case optionally in the form of a corresponding N-oxide, a corresponding salt or a corresponding solvate thereof.

The other active component of the inventive active substance combination, component (B), is an anti-addictive agent.

The term "anti-addictive agent" as used herein denotes any compound that is useful to prevent or at least ameliorate addiction or symptoms resulting from addiction.

Preferably component (B) is an anti-alcohol-addictive agent, an anti-drug-addictive agent or an anti-nicotine-addictive agent. However, those skilled in the art recognize that there may be an overlap between the different groups of anti-addictive agents, i.e. an anti-nicotine-addictive agent may also be useful in the prevention or amelioration of drug addiction. Also, two or more agents from the same class of anti-addictive agents may be used in the inventive active substance combination. Two or more anti-addictive agents of one and the same class may also be present in the inventive active substance combination.

The term "Drug" as used herein includes all drugs that have a potential for abuse and/or addiction. A comprehensive list of such drugs is, for example, provided by the US Department of Health and Human Services /National Clearinghouse for Alcohol and Drug Information (NCADI).

Anti-addictive agents (Drugs used in addictive disorders), such as anti-alcohol-addictive agents (Drugs used in alcohol-dependence), anti-drug-addictive agents (Drugs used in drug dependence) or anti-nicotine-addictive agents (Drugs used in nicotine dependence) are well known to those skilled in the art, e.g. from the ATC-index edited by the World Health Organisation.

The anti-alcohol-addictive agent may preferably be selected from the group consisting of Melperone, Nitrefazole, Disulfiram, Diazepam, Carbamazepine, Clobazam, Tiapride, Naltrexone, Vigabatrin, Acamprosate, Ondansetron, Nalmefene, Aripiprazole, Sodium oxybate, Rimonabant, Tryptosine (Triptosine), Ecopipam, Body protection compound-15, Ritanserin, Neramexane, Agmatine, Naltriben, Costunolide, Dehydrocostus lactone and Epipregnanolone, optionally in the form of a corresponding salt or a corresponding solvate thereof.

More preferably, the anti-alcohol-addictive agent may be selected from the group consisting of Melperone, Nitrefazole, Disulfiram, Diazepam, Carbamazepine, Clobazam, Tiapride, Naltrexone, Vigabatrin, Acamprosate, Ondansetron, Nalmefene, Aripiprazole, Sodium oxybate and Rimonabant, optionally in the form of a corresponding salt or a corresponding solvate thereof.

The anti-drug-addictive agent may preferably be selected from the group consisting of Disulfiram, Buprenorphine, Selegiline, Vigabatrin, Cabergoline, Nalmefene, Methadone, Naltrexone, Lofexidine, Amineptine, Flumazenil, Flumazepil, L-Deprenyl, Vanoxerine, Ecopipam, Adrogolide, Cocaine-BSA conjugate and Cocaine conjugate vaccine, optionally in the form of a corresponding salt or a corresponding solvate thereof.

More preferably, the anti-drug-addictive agent may be selected from the group consisting of Disulfiram, Buprenorphine, Selegiline, Vigabatrin, Cabergoline, Nalmefene, Methadone, Naltrexone, Lofexidine, Amineptine, Flumazenil and Flumazepil, optionally in the form of a corresponding salt or a corresponding solvate thereof.

The anti-nicotine-addictive agent may preferably be selected from the group consisting of Mecamylamine, Naltrexone, Anabasine, Amfebutamone; Bupropion, Topiramate, Rimonabant, Nicotine conjugate vaccine, Befloxatone, Physostigmine/Scopolamine, Cotinine Qb-Nicotine, Nicotine-Qbeta, Lazabemide, (L)-Lobeline and Varenicline, optionally in the form of a corresponding salt or a corresponding solvate thereof.

More preferably, the anti-nicotine-addictive agent may be selected from the group consisting of Mecamylamine, Naltrexone, Anabasine, Amfebutamone; Bupropion, Topiramate and Rimonabant, optionally in the form of a corresponding salt or a corresponding solvate thereof.

More preferred is an inventive active substance combination comprising

(A) at least one substituted pyrazoline compound of general formula I

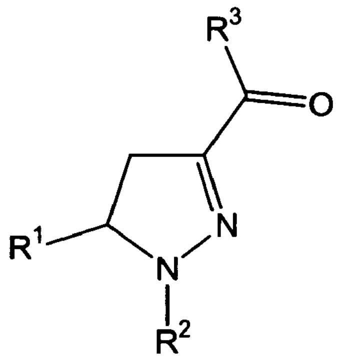

I

wherein

$R^1$ represents a phenyl group, which is optionally substituted with 1, 2, 3, 4 or 5 substituents independently selected from the group consisting of methyl, ethyl, F, Cl, Br and $CF_3$,

$R^2$ represents a phenyl group, which is optionally substituted with 1, 2, 3, 4 or 5 substituents independently selected from the group consisting of methyl, ethyl, F, Cl, Br and $CF_3$,

$R^3$ represents a pyrrolidinyl group, a piperidinyl group or a piperazinyl group, whereby each of these groups may be substituted with one or more of $C_{1-6}$-alkyl groups, or $R^3$ represents an -$NR^4R^5$-moiety,

$R^4$ represents a hydrogen atom or a linear or branched $C_{1-6}$-alkyl group,

$R^5$ represents a linear or branched $C_{1-6}$ alkyl group; an -$SO_2$-$R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and

$R^6$ represents a phenyl group, which is optionally substituted with one or more $C_{1-6}$ alkyl groups, which may be identical or different.

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,

and/or

at least one substituted pyrazoline compound of general formula I' represented by the following structure II:

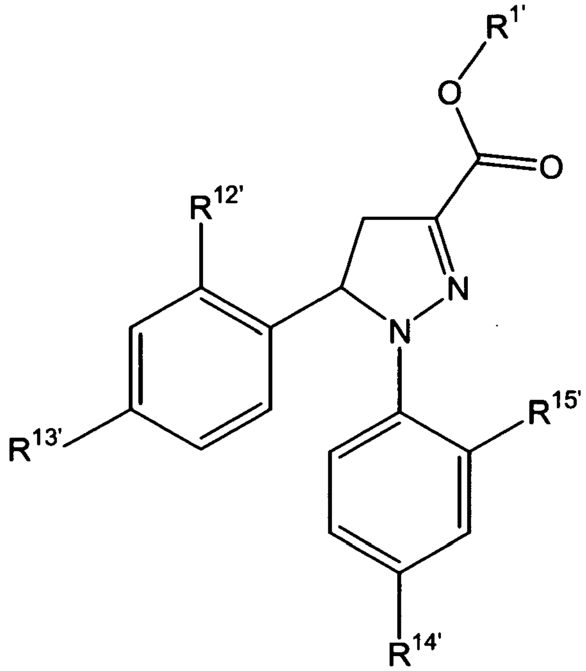

II

wherein

$R^{1'}$ represents hydrogen or a linear or branched $C_{1-4}$-alkyl group,

$R^{12'}$ or $R^{13'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,

$R^{14'}$ or $R^{15'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

and

(B) (B)at least one anti-addictive agent selected from the group consisting of an anti-alcohol-addictive agent, an anti-drug-addictive agent and an anti-nicotine-addictive agent.

Yet more preferred is an inventive active substance combination comprising

one or more substituted pyrazoline compounds of general formula I selected from the group consisting of

N-piperidinyl-5-(4-chloro-phenyl)-1-(2,4-dichlorophenyl)-4,5-dihydro-1H-pyrazol-3-carboxamide,

5-(4-Chloro-phenyl)-1-(2,4-dichloro-phenyl)-4,5-dihydro-1H-pyrazole-3-carboxylic acid-[1,2,4]-triazole-4-yl-amide,

5-(4-Chloro-phenyl)-1-(2,4-dichloro-phenyl)-4,5-dihydro-1H-pyrazole-3-carboxylic acid-(4-methyl-piperazin-1-yl)-amide,

5-(4-Chloro-phenyl)-1-(2,4-dichloro-phenyl)-4,5-dihydro-1H-pyrazole-3-carboxylic acid diethylamide,

[5-(4-Chloro-phenyl)-1-(2,4-dichloro-phenyl)-4,5-dihydro-1H-pyrazole-3-yl]-piperidine-1-yl-methanone and

N-[5-(4-Chloro-phenyl)-1-(2,4-dichlorophenyl)-4,5-dihydro-1H-pyrazole-3-carbonyl]-4-methylphenylsulfonamide,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,

and/or

5-(4-chloro-phenyl)-1-(2,4-dichlorophenyl)-4,5-dihydro-1H-pyrazol-3-carboxylic acid

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof,

and

at least one anti-addictive agent selected from the group consisting of an anti-alcohol-addictive agent, an anti-drug-addictive agent and an anti-nicotine-addictive agent.

The inventively used substituted pyrazoline compounds may, for example, be obtained by the following process, according to which which at least one benzaldehyde compound of general formula II

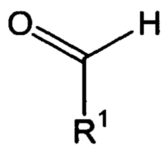

(II)

wherein $R^1$ has the meaning given above, is reacted with a pyruvate compound of general formula (III)

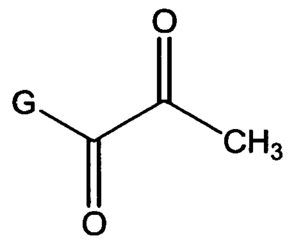

(III),

wherein G represents an OR group with R being a branched or unbranched $C_{1-6}$ alkyl radical, preferably an ethyl radical, or G represents an $O^-K$ group with K being a cation, preferably a monovalent cation, more preferably an alkali metal cation, even more preferably a sodium cation, to yield a compound of general formula (IV)

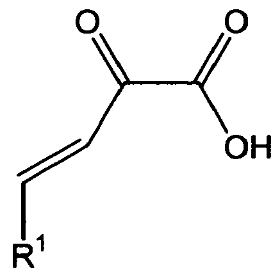

(IV)

wherein $R^1$ has the meaning given above, which is optionally isolated and/or optionally purified, and which is reacted with an optionally substituted phenyl hydrazine of general formula (V)

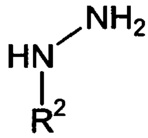

(V)

or a corresponding salt thereof, wherein $R^2$ has the meaning given above, under an inert atmosphere, to yield a compound of general formula (VI)

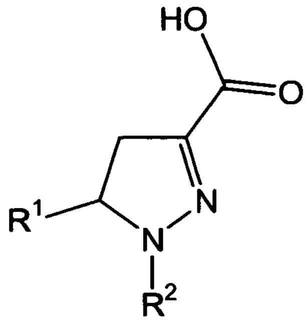

(VI)

wherein $R^1$ and $R^2$ have the meaning as given above, which is optionally isolated and/or optionally purified, and optionally transferred under inert atmosphere to a compound of general formula (VII)

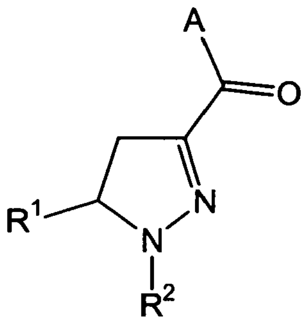

(VII)

wherein the substituents $R^1$ and $R^2$ have the meaning given above and A represents a leaving group, via the reaction with an activating agent, said compound being optionally isolated and/or optionally purified, and at least one compound of general formula (VI) is reacted with a compound of general formula $R^3H$, wherein $R^3$ represents an $-NR^4R^5$-moiety, wherein $R^4$ and $R^5$ have the meaning given above, to yield a substituted pyrazoline compound of general formula I, wherein $R^3$ represents an $-NR^4R^5$-moiety,
and/or at least one compound of general formula (VII) is reacted with a compound of the general formula $R^3H$, in which

$R^3$ has the meaning given above to yield a compound of general formula (I) given above, which is optionally isolated and/or optionally purified.

The process is also illustrated in scheme I given below:

**Scheme I:**

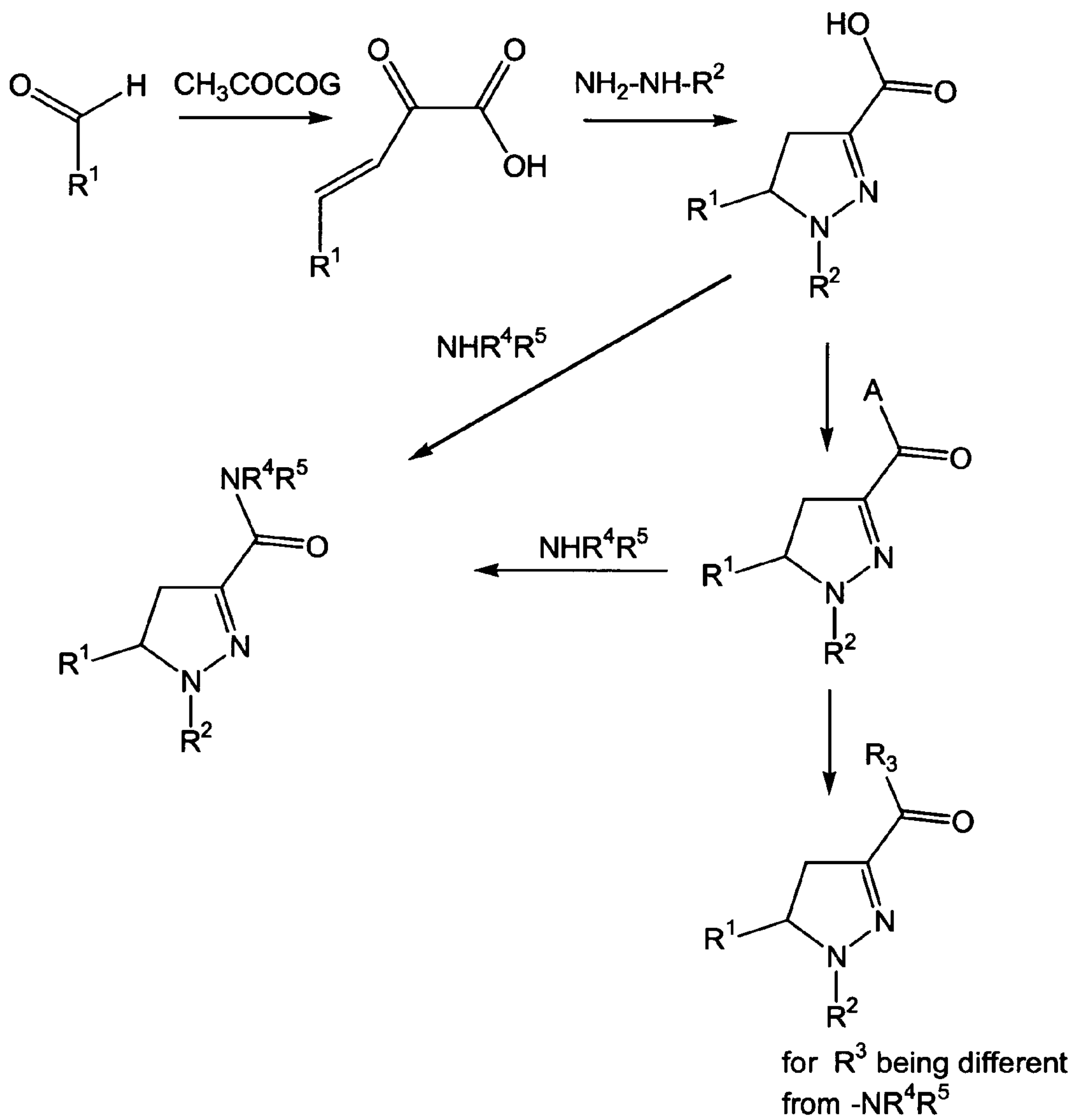


The reaction of the benzaldehyde compound of general formula II with a pyruvate compound of general formula III is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula II with a pyruvate compound of general formula III is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (IV) with an optionally substituted phenyl hydrazin of general formula (V) is preferably carried out in a suitable reaction medium such as $C_{1-4}$-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VI) may be activated for further reactions by the introduction of a suitable leaving group according to conventional methods well known to those skilled in the art. Preferably the compounds of general formula (VI) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (VII) is an acid chloride, it is preferably prepared by reaction of the corresponding acid of general formula (VI) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0° C to the boiling point of the solvent and reaction times from several minutes to several hours.

If said activated compound of general formula (VII) is a mixed anhydride, said anhydride may preferably be prepared, for example, by reaction of the corresponding acid of general formula (VI) with ethyl chloroformiate in the presence of a base such as triethylamine or pyridine, in a suitable solvent.

The reaction of general formula (VII) with a compound of general formula $HR^3$ to yield compounds of general general I, wherein $R^3$ represents an $-NR^4R^5$ moiety is preferably carried out in presence of a base such as triethylamine in a reaction medium such as methylenchloride. The temperature is preferably in the range from 0°C to the boiling point of the reaction medium. The reaction time may vary over a broad range, e.g. from several hours to several days.

The reaction of general formula (VII) with a compound of general formula $HR^3$ to yield compounds of general formula I, wherein $R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system may be carried out according to conventional methods well known to those skilled in the art, e.g. from Pascual, A., J. Prakt Chem., 1999, 341(7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, tetrahydrofurane or similar. The temperature is preferably in teh range from 0°C to the boiling point of the reaction medium, more preferably from 15 to 25 °C. The reaction time may vary over a broad range, e.g. from several minutes to several hours.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula (I) themselves are obtained in form of a mixture of stereoisomers, particularly enantiomers or diastereomers, said mixtures may be separated by standard procedures known to those skilled in the art, e.g. chromatographic methods or fractunalized crystallization with chiral reagents. It is also possible to obtain pure stereoisomers via stereoselective synthesis.

Salts of the inventively used active compounds may be obtained by a process, wherein at least one of the compounds having at least one basic group is reacted with at least one inorganic and/or organic acid, preferably in the presence of a suitable reaction medium. Suitable reaction media include, for example, any of the ones given above.

Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

Salts of the inventively used active compounds may also be prepared by a method, wherein at least one of the compounds having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

Solvates, preferably hydrates, of the inventively used substituted pyrazoline compounds of general formula (I), of corresponding stereoisomers, of corresponding N-oxides or of corresponding salts thereof may also be obtained by standard procedures known to those skilled in the art.

Substituted pyrazoline compounds of general formula I, which comprise nitrogen-atom containing saturated, unsaturated or aromatic rings may also be obtained in the form of their N-oxides by methods well known to those skilled in the art and used in the active substance combination of the present invention.

Those skilled in the art understand that the term substituted pyrazoline compounds as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

The purification and isolation of the inventively used substituted pyrazoline compounds of general formula (I), of a corresponding stereoisomer, or salt, or N-oxide, or solvate or any intermediate thereof may, if required, be carried out by conventional methods known to those skilled in the art, e.g. chromatographic methods or recrystallization.

The inventive active substance combination may comprise the components (A) and (B) in a molar ratio of component (A) to component (B) in the range of 1:10 to 10:1, preferably 1:5 to 5:1.

The inventive active substance combination is suitable for the administration to humans, including infants, children and grown-ups, as well as animals.

Preferably the total amount of the active substance(s) according to component (A), calculated as the free compound(s), to be administered to the patient in a 24 hours period does not exceed 800 mg, preferably does not exceed 500 mg.

The total amount of the active substance(s) according to component (B), calculated as the free compound(s), to be administered to the patient in a 24 hours period does preferably not exceed 160 mg, more preferably does not exceed 80 mg. Preferably the inventive active substance combination comprises components (A) and (B) in the above defined molar ratios and within the afore given limits for the maximum dosis to be administered per day. Medicaments on the basis of the inventive active substance combination may preferably be administered once daily, twice daily or three times daily, more preferably once daily or twice daily, most preferably once daily.

In another aspect the present invention relates to a medicament comprising an inventive active substance combination and optionally at least one further active substance and/or optionally at least one auxiliary substance.

Specifically, the inventive medicament is suitable for the modulation of cannabinoid-receptors, preferably cannabinoid 1 ($CB_1$) receptors; and/or for HMG-CoA reductase inhibition.

The inventive medicament is particular useful for the prophylaxis and/or treatment of coronary heart disease, myocardial ischemia, angina pectoris, atherosclerosis, hypertension, hyperlipidemia, Lipoprotein disorders, Hypercholesterolemia, or for lowering of cardiac risk.

Cardiac risk as used herein means that a subject (human or animal) will suffer a future adverse cardiac event such as, e.g. myocardial infarction, cardiac arrest, cardiac failure, cardiac ischemia. Cardiac risk can be calculated using the Framingham Risk equation, as disclosed with respect to the Framingham Heart Study, in Wilson et al., Am. J. Cardiol. 1987, 59(14):91G-94G. The respective part of the literature is herewith incorporated by reference and forms part of the present disclosure.

The inventive medicament is also suitable for the modulation of cannabinoid-receptors, preferably cannabinoid 1 ($CB_1$) receptors; for the regulation of triglyceride levels in the blood plasma; for the prophylaxis and/or treatment of disorders of the central nervous system; for the prophylaxis and/or treatment of disorders of the cardiovascular system; for the prophylaxis and/or treatment of disorders of the immune system; for the prophylaxis and/or treatment of disorders of the endocrinous system; for the prophylaxis and/or treatment of disorders of the respiratory system; for the prophylaxis and/or treatment of disorders of the gastrointestinal tract and/or for the prophylaxis and/or treatment of reproductive disorders.

Moreover, the inventive medicament is also suitable for the prophylaxis and/or - treatment of one or more of the following disorders:

food intake disorders, preferably selected from the group consisting of bulimia; anorexia; cachexia; obesity and type II diabetes mellitus (non-insuline dependent diabetes mellitus), more preferably obesity; psychosis; alcohol abuse and/or addiction; nicotine abuse and/or addiction; drug abuse and/or addiction; medicament abuse and/or addiction; schizophrenia; anxiety; depression; epilepsy; neurodegenerative disorders, preferably Morbus Parkinson; Morbus Huntington; Morbus Alzheimer and/or Multiple Sclerosis; cerebellar disorders; spinocerebellar disorders; cognitive disorders; cranial trauma; panic attacks; peripheric neuropathy; glaucoma; migraine; Raynaud's disease; tremblement disorders; compulsive disorders; senile dementia; thymic disorders; tardive dyskinesia; bipolar disorders; bone disorders including osteoporosis or Paget's disease of bone; cancer, preferably for the prophylaxis and/or treatment of one or more types of cancer selected from the group consisting of brain cancer; bone cancer; lip cancer; mouth cancer; esophageal cancer; stomach cancer; liver cancer; bladder cancer; pancreas cancer; ovary cancer; cervical cancer; lung cancer; breast cancer; skin camcer; colon cancer; bowl cancer and prostate cancer, more preferably for the prophylaxis and/or treatment of one or more types of cancer selected from the group consisting of colcon cancer; bowel cancer and prostate cancer, medicament-induced movement disorders; dystonia; endotoxemic shock; stroke; hemorragic shock; hypotension; insomnia; immunologic disorders; sclerotic plaques; vomiting; diarrhea; asthma; memory disorders; pruritus; pain; or for potentiation of the analgesic effect of narcotic and non-narcotic analgesics; or for influencing intestinal transit.

Those skilled in the art understand that the components (A) and (B) of the active substance combination as well as the different components of components (A) may be administered simultaneously or sequentially to one another, whereby in each case components (A) (including one or both of substituted pyrazoline compounds of general formula I and I') and (B) may be administerd via the same or different administration pathways, e.g. orally or parenterally. preferably both components (A) and (B) are administered simultaneously in one and the same administration form.

Another aspect of the present invention relates to the use of an inventive pharmacologically active substance combination for the preparation of a medicament for the modulation of cannabinoid-receptors, preferably cannabinoid 1 ($CB_1$) receptors; and/or for HMG-CoA reductase inhibition.

Another aspect of the present invention relates to the use of an inventive pharmacologically active substance combination for the preparation of a medicament for the prophylaxis and/or treatment of coronary heart disease, myocardial ischemia, angina pectoris, atherosclerosis, hypertension, hyperlipidemia, Lipoprotein disorders, Hypercholesterolemia, or for lowering of cardiac risk.

Another aspect of the present invention relates to the use of an inventive pharmacologically active substance combination for the preparation of a medicament for the modulation of cannabinoid-receptors, preferably cannabinoid 1 ($CB_1$) receptors; for the regulation of triglyceride levels in the blood plasma; for the prophylaxis and/or treatment of disorders of the central nervous system; for the prophylaxis and/or treatment of disorders of the cardiovascular system; for the prophylaxis and/or treatment of disorders of the immune system; for the prophylaxis and/or treatment of disorders of the endocrinous system; for the prophylaxis and/or treatment of disorders of the respiratory system; for the prophylaxis and/or treatment of disorders of the gastrointestinal tract and/or for the prophylaxis and/or treatment of reproductive disorders.

The use for the preparation of a medicament for the prophylaxis and/or treatment of one or more of the following disorders:

food intake disorders, preferably selected from the group consisting of bulimia; anorexia; cachexia; obesity and type II diabetes mellitus (non-insuline dependent diabetes mellitus), more preferably obesity; psychosis; alcohol abuse and/or addiction; nicotine abuse and/or addiction; drug abuse and/or addiction; medicament abuse and/or addiction; schizophrenia; anxiety; depression; epilepsy; neurodegenerative disorders, preferably Morbus Parkinson; Morbus Huntington; Morbus Alzheimer and/or Multiple Sclerosis; cerebellar disorders; spinocerebellar disorders; cognitive disorders; cranial trauma; panic attacks; peripheric neuropathy; glaucoma; migraine; Raynaud's disease; tremblement disorders; compulsive disorders; senile dementia; thymic disorders; tardive dyskinesia; bipolar disorders; bone disorders including osteoporosis or Paget's disease of bone; cancer, preferably for the prophylaxis and/or treatment of one or more types of cancer selected from the group consisting of brain cancer; bone cancer; lip cancer; mouth cancer; esophageal cancer; stomach cancer; liver cancer; bladder cancer; pancreas cancer; ovary cancer; cervical cancer; lung cancer; breast cancer; skin camcer; colon cancer; bowl cancer and prostate cancer, more preferably for the prophylaxis and/or treatment of one or more types of cancer selected from the group consisting of colcon cancer; bowel cancer and prostate cancer, medicament-induced movement disorders; dystonia; endotoxemic shock; stroke; hemorragic shock; hypotension; insomnia; immunologic disorders; sclerotic plaques; vomiting; diarrhea; asthma; memory disorders; pruritus; pain; or for potentiation of the analgesic effect of narcotic and non-narcotic

analgesics; or for influencing intestinal transit

is particularly preferred.

A further aspect of the present invention relates to pharmaceutical formulations in different pharmaceutical forms comprising an inventive active substance combination and optionally at least one further active substance and/or optionally at least one auxiliary substance.

Preferably the inventive pharmaceutical formulation is suitable for oral or parenteral administration, more preferably for oral, intravenous, intraperitoneal, intramuscular, subcutaneous, intrathekal, rectal, transdermal, transmucosal or nasal administration.

Inventive pharmaceutical formulation for oral administration are preferably selected from the group consisting of tablets, drageés, capsules, powders, drops, gels, juices, sirups, solutions and suspensions.

The pharmaceutical formulation of the present invention for oral administration may also be in the form of multiparticulates, preferably microparticles, microtablets, pellets or granules, optionally compressed into a tablet, filled into a capsule or suspended in a suitable liquid. Suitable liquids are known to those skilled in the art.

The respective pharmaceutical formulations may - depending on their route of administration - also contain one or more auxiliary substances known to those skilled in the art. The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the present disclosure.

In one embodiment of the present invention the pharmaceutical formulation comprises one or both of the components (A) and (B) at least partially in a sustained-release form. Preferably the inventive pharmaceutical formulation comprises component (B) at least partially in a sustained-release form.

By incorporating one or both of these components at least partially or completely in a sustained-release form it is possible to extend the duration of their effect, allowing for the beneficial effects of such a sustained release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.

The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly($C_{1-4}$)alkyl (meth) acrylates, poly($C_{1-4}$)dialkylamino($C_{1-4}$)alkyl (meth)acrylates and/or copolymers or mixtures thereof, and very particularly preferably copolymers of ethyl acrylate and methyl methacrylate with a monomer molar ratio of 2:1 (Eudragit NE30D®), copolymers of ethyl acrylate, methyl methacrylate and trimethylammonium ethyl methacrylate-chloride with a monomer molar ratio of 1:2:0.1 (Eudragit RS®), copolymers of ethyl acrylate, methyl methacrylate and trimethylammonium ethyl methacrylate-chloride with a monomer molar ratio of 1:2:0.2 (Eudragit RL®), or a mixture of at least two of the above-mentioned copolymers. These coating materials are commercially available as 30 wt.% aqueous latex dispersions, i.e. as Eudragit RS30D®, Eudragit NE30D® or Eudragit RL30D®, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat® or Surelease®.

As natural, semisynthetic or synthetic waxes, fats or fatty alcohols, the sustained-release material may be

based on camauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a $C_6$-$C_{40}$ aliphatic or aromatic dicarboxylic acid and a $C_1$-$C_8$ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet® (acetylated mono- and diglycerides, $C_{23}H_{44}O_5$ to $C_{25}H_{47}O_7$), medium-chain triglycerides (Miglyol®), oleic acid or mixtures of at least two of said plasticizers. Aqueous dispersions of Eudragit RS® and optionally Eudragit RL® preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) covered by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L®), methacrylic acid/methyl methacrylate copolymers with a monomer molar ratio of 1:2 (Eudragit S®), methacrylic acid/ethyl acrylate copolymers with a monomer molar ratio of 1:1 (Eudragit L30D-55®), methacrylic acid/methyl acrylate/methyl methacrylate copolymers with a monomer molar ratio of 7:3:1 (Eudragit FS®), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D® and/or Eudragit RL® and/or Eudragit RS®.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1, 299-311. The respective descriptions are incorporated by reference and are part of the present disclosure.

In another embodiment, the pharmaceutical formulation of the present invention contains one or both of components (A) and (B) not only in sustained-release form, but also in non-retarded form. By combination with the immediately released form, a high initial dose can be achieved for the rapid onset of the beneficial effect. The slow release from the sustained release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

**Pharmacological Methods**

**I. In-vitro determination of affinity to CB1/CB2-Receptors**

The in-vitro determination of the affinity of the substituted pyrazoline compounds to $CB_1$/$CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description is hereby incorporated by reference and forms part of the present disclosure.

**II. In-vivo bioassay system for determination of cannabinoid activity**

**Mouse tetrad model**

Substances with affinity for cannabinoid receptors are known to produce a wide range of pharmacological

effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, Intemational Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

**Material and Methods**

Male NMRI mice with a weight of 20-30 g (Harlan, Barcelona, Spain) are used in all of the following experiments.

Before testing in the behavioral procedures given below, mice are acclimatized to the experimental setting. Pre-Treatment control values are determined for analgesia hot plate latency (in seconds), rectal temperature, sedation and catalepsy.

In order to determine the agonistic activty of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

**Hot plate analgesia**

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 ± 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minuts after the administration of the substance to be tested, the mice are again placed on the hot plate and the afore described procedure is repeated. This period is called the post-treatment reading (PT).

The degree of analgesia is calculated from the formula :

$$\% \text{ MPE of Analgesia} = (PT - B) / (PC - B) \times 100$$

MPE = Maximum possible effect.

**Determination of sedation and ataxia**

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.

The chosen scoring system is

0: no ataxia;
1: doubful;
2: obvious calmness and quiet;
3 pronounced ataxia;

prior to as well as after treatment.

The percentage of sedation is determined according to the formula:

$$\% \text{ of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is calculated for each animal, whereby differences of ≥-2 °C are considered to represent activity.

**Catalepsy:**

Catalepsy is determined according to the method described in Alpermann H. G. et al. "Pharmacological effets of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:
more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according ot the following formula:

$$\% \text{ Catalepsy} = \text{arithmetic mean} / 6 \times 100$$

The present invention is illustrated below with the aid of examples. These illustrations are given solely by way of example and do not limit the general spirit of the present invention.

**Examples:**

**Example 1:**

**N-piperidinyl-5-(4-chlorophenyl)-1-(2,4-dichlorophenyl)-4,5-dihydro-1H-pyrazole-3-carboxamide**

**a) 4-(4-chlorophenyl)-2-oxo-3-butenoic acid**

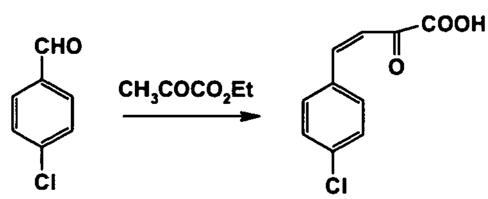

In a three neck flask p-chlorobenzaldehyde (13,3 g, 95 mmoles) and ethyl pyruvate (10 g, 86 mmoles) were dissolved in 150 ml of absolute ethanol. The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).
IR (KBr, $cm^{-1}$) : 3500-2500, 1719,3, 1686,5, 1603,4, 1587,8, 1081,9.
$^1$H NMR($CDCl_3$, δ) : 7,4 (d, J=8,4Hz, 2H), 7,5 (d, J=16,1Hz, 1 H), 7,6 (d, J=8,4Hz, 2H), 8,1(d, J=16,1 Hz, 1 H).

**b) 5-(4-chlorophenyl)-1-(2,4-dichlorophenyl)-4,5-dihydro-pyrazole-3-carboxylic acid**

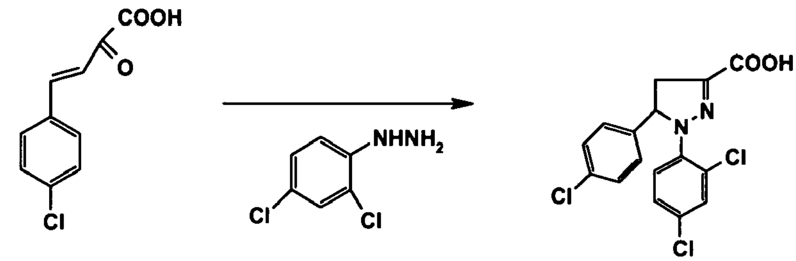

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).
IR (KBr, $cm^{-1}$): 3200-2200, 1668,4, 1458, 1251,4, 1104,8.
$^1$H NMR ($CDCl_3$, δ) : 3,3 (dd, 1 H), 3,7 (dd, 1 H), 5,9 (dd, 1 H), 7,09-7,25 (m, 7H).

**(c) 5-(4-chlorophenyl)-1-(2,4-dichlorophenyl)-4,5-dihydro-pyrazole-3-carboxylic acid chloride**

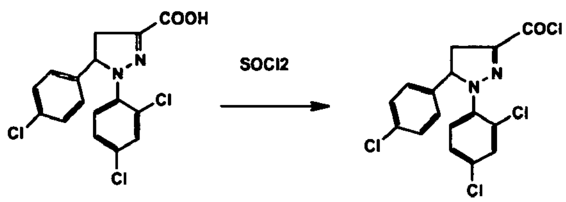


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.
IR (KBr, $cm^{-1}$) : 1732,3, 1700, 1533,3, 1478,1, 1212,9, 826,6.

**d) N-piperidinyl-5-(4-chlorophenyl)-1-(2,4-dichlorophenyl)-4,5-dihydropyrazole-3-carboxamide** [this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

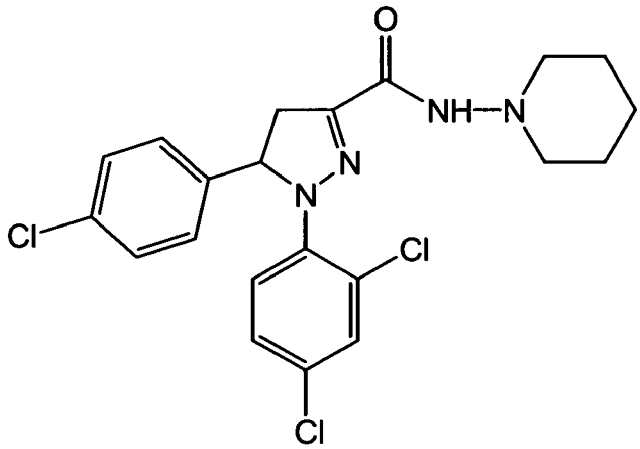

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.
IR (KBr, $cm^{-1}$) : 3222,9, 2934,9, 1647,4, 1474,7, 1268,3, 815,6.
$^1H$ NMR ( $CDCl_3$, δ) : 1,4 (m, 2H), 1,7 (m, 4H), 2,8 (m, 4H), 3,3 (dd, J=6,1 y 18,3Hz, 1H), 3,7 (dd, J=12,5 and 18,3 Hz, 1H), 5,7 (dd, J=6,1 and 12,5 Hz, 1H), 7,0-7,2 (m, 6H), 7,4(s, 1H).

The compounds according to the following examples 2-6 have been prepared analogously to the process described in Example 1.

**Example 2:**

**5-(4-Chloro-phenyl)-1-(2,4-dichloro-phenyl)-4,5-dihydro-1H-pyrazole-3-carboxylic acid-[1,2,4]triazol-4-yl amide**

Melting point: 134-138 °C.
IR (KBr, $cm^{-1}$): 3448, 1686, 1477, 1243, 1091, 821.
$^1H$ NMR($CDCl_3$, δ): 3,1 (dd, J=6,2 and 17,9Hz, 1 H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1H).

**Example 3:**

**5-(4-Chloro-phenyl)-1-(2,4-dichloro-phenyl)-4,5-dihydro-1H-pyrazole-3-carboxylic acid-(4-methyl-piperazin-1-yl)-amide hydrochloride**

Melting point: 150-155°C.
IR (KBr, $cm^{-1}$) : 3433, 1685, 1477, 1296, 1246, 1088, 1014, 825.

$^1H$ NMR ($CDCl_3$, δ): 2,7 (d, J=4,2Hz, 3H), 3,0-3,4 (m, 9H), 3,6 (dd, J=11,9 and 17,9 Hz, 1H), 5,8 (dd, J=5,5 and 11,9 Hz, 1H), 7,1 (d, J=8,4Hz, 2H), 7,25 (2d, J= 8,4 and 8,7 Hz, 3H), 7,4 (d, J=2,2Hz, 1H), 7,5 (d, J=8,7Hz, 1H), 9,8 (s, 1H), 11,2 (bs).

**Example 4:**

**5-(4-Chloro-phenyl)-1-(2,4-dichloro-phenyl)-4,5-dihydro-1**H-pyrazole-3-**carboxylic acid diethylamide**

This compound was obtained in form of an oil.
IR (film, $cm^{-1}$) : 2974, 1621, 1471, 1274, 1092, 820.
$^1H$ NMR ($CDCl_3$, δ): 1,2 (m, 6H), 3,3-3,9 (m, 6H), 5,6 (dd, J=5,8 and 11,7 Hz, 1H), 7-7,25 (m, 7H).

**Example 5:**

**[5-(4-Chloro-phenyl)-1-(2,4-dichloro-phenyl)-4,5-dihydro-1H-pyrazol-3-yl]-piperidin-1-yl-methanone**

Melting point: 105-110°C.
IR (KBr, $cm^{-1}$) : 2934, 1622, 1470, 1446, 1266, 1010, 817.
$^1H$ NMR ($CDCl_3$, δ): 1,7 (m, 6H), 3,4 (dd, J=5,7 and 17,9Hz, 1H), 3,7 (m, 3H), 3,9 (m, 2H), 5,6 (dd, J=6,1 y 11,9 Hz, 1H), 7-7,25 (m, 7H).

**Example 6:**

**N-[5-(4-Chloro-phenyl)-1-(2,4-dichloro-phenyl)-4,5-dihydro-1H-pyrazole-3-carbonyl]-4-methyl-phenylsulfonamide**

This compound was obtained in form of an amorph solid.
IR (KBr, $cm^{-1}$) : 1697, 1481, 1436, 1340, 1169, 1074, 853.
$^1H$ NMR ($CDCl_3$, δ): 2,4 (s, 3H), 3,2 (dd, J=6,6 and 18,3Hz, 1 H), 3,6 (dd, J=12,8 and 18,3Hz, 1 H), 5,8 (dd, J=6,6 and 12,8Hz, 1H), 7 (d, J=8,2Hz, 2H), 7,2 (s, 1 H), 7,3-7,4 (m, 6H), 8 (d, J=8,1 Hz, 2H), 9 (s, 1 H).

**Example 7:**

N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 °C and m-chloroperbenzoic acid (0,204 g, 0,83 mmoles) added in several portions. After stirring for 15 minutes a control via thin layer chromatography showed that no starting material was remaining. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120°C.
IR (KBr, $cm^{-1}$): 3202, 1678, 1654, 1474, 1309, 1107.
$^1H$-NMR ($CDCl_3$, δ): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1 H), 7.0-7.3 (m, 7H), 8.5(s, 1H.)

**Pharmacological Data:**

**I. In-vitro determination of affinity to $CB_1/CB_2$-Rezeptors**

The affinity of the inventive substituted pyrazoline compounds to $CB_1/CB_2$ receptors was determined as described above. Some of the values obtained are given in the following table I:

**Table I:**

| Compound according to Example | $CB_1$-Receptor Radiologand:[$^3$H]-CP55940 | | $CB_2$-Receptor Radiologand:[$^3$H]-CP55940 | |
|---|---|---|---|---|
| | % Inhibition $10^{-6}$ M | $K_i$(nM) | % Inhibition $10^{-6}$ M | $K_i$(nM) |
| 1 | 93% | <25 | 33% | > 1000 |
| 5 | 79% | 111 | 54% | ≈ 1000 |

As can be seen from the values given in table 1 the inventive pyrazoline compounds are particularly suitable for regulating the $CB_1$-Receptor.

**II. In-vivo bioassay system for determination of cannabinoid activity**

The determinination of cannabinoid activity in-vivo was determined as described above. Some of the values obtained are given in the following table II:

Table II:

| Compound according to example : | dosis administered: 5 mg/kg i.v. Agonistic effect | | | | dosis administered: 5 mg/kg i.v. prior to Win 55212-2 in a dose of 1,25 mg/kg i.v. Antagonistic Effect | | | |
|---|---|---|---|---|---|---|---|---|
| | A | B | C. | D | A | B. | C | D |
| 1 | 0 | 0 | 0 | 0 | 74 | 100 | 100 | 100 |
| 5 | 0 | 50 | 0 | 0 | 50 | 40 | 20 | 20 |
| i.v. intravenous<br>A: Hot-Plate test<br>B: Hypothermia<br>C: Catalepsy<br>D: Sedation | | | | | | | | |

As can be seen from the values given in table II the inventive pyrazoline compounds show an antagonistic effect.

## Claims

**1.** Active substance combination comprising

(A) at least one substituted pyrazoline compound of general formula I

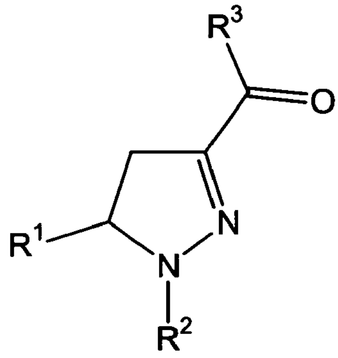

I

wherein

$R^1$ represents an optionally at least mono-substituted phenyl group;

$R^2$ represents an optionally at least mono-substituted phenyl group;

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an —$NR^4R^5$-moiety,

$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group; an — $SO_2$-$R^6$-moiety; or an -$NA^7A^8$-moiety, with the proviso that $R^4$ and $R^5$ do not identically represent hydrogen;

$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono- or polycyclic ring system and/or bonded via a linear or branched alkylene group;

$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and/or

at least one substituted pyrazoline compound of general formula I',

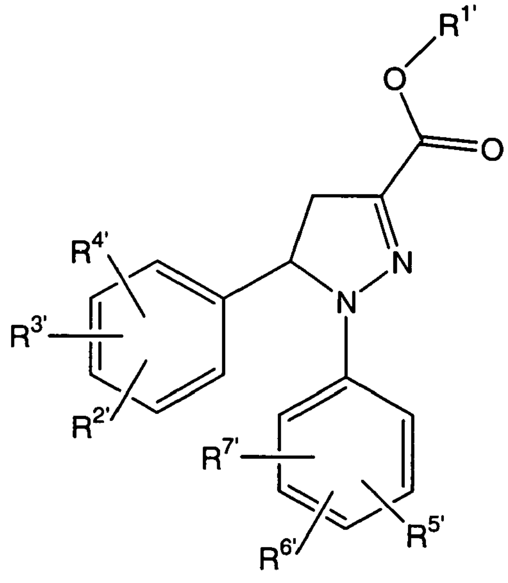

I'

wherein
$R^{1'}$ represents hydrogen or a linear or branched $C_{1-4}$-alkyl group,
$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{8'}$, SH, $SR^{8'}$, $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched $C_{1-6}$ alkyl,
$R^{5'}$ and $R^{6'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
$R^{7'}$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
and
(B) at least one anti-addictive agent.

2. Active substance combination according to claim 1, **characterized in that** $R^1$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R" whereby R' and R" for each substituent independently represent linear or branched $C_{1-6}$ alkyl, preferably $R^1$ represents a phenyl group, which is optionally substituted by one or more substituents selected from the group consisting of methyl, ethyl, F, Cl, Br and $CF_3$, more preferably $R^1$ represents a phenyl group, which is mono-substituted with a chlorine atom in the 4-position.

3. Active substance combination according to claim 1 or 2, **characterized in that** $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and optionally R" for each substituent independently represent linear or branched $C_{1-6}$ alkyl, preferably $R^2$ rep-

resents a phenyl group, which is optionally substituted by one or more substituents independently selected from the group consisting of methyl, ethyl, F, Cl, Br and $CF_3$, more preferably $R^2$ represents a phenyl group, which is disubstituted with two chlorine atoms in its 2- and 4-position.

**4.** Active substance combination according to one or more of claims 1-3, **characterized in that** $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an —$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an -$NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an —$NR^4R^5$-moiety.

**5.** Active substance combination according to one or more of claims 1-4, **characterized in that** $R^4$ and $R^5$, identical or different, represent a hydrogen atom; an unbranched or branched, saturated or unsaturated, optionally at least mono-substituted $C_{1-6}$-aliphatic radical; a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system and/or bonded via a methylene ($-CH_2-$) or ethylene ($-CH_2-CH_2$)-group; an -$SO_2$-$R^6$-moiety; or an -$NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring member containing $C_{3-8}$-cydoaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system; an -$SO_2$-$R^6$-moiety; or an -$NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an —$SO_2$-$R^6$-moiety; or an -$NR^7R^8$-moiety, or $R^4$ and $R^5$, identical or different, represent a methyl group, an ethyl group, an n-propyl group, an isopropyl group, an n-butyl group, a sec-butyl group or a tert.-butyl group.

**6.** Active substance combination according to one or more of claims 1-5, **characterized in that** $R^6$ represents a linear or branched, saturated or unsaturated, optionally at least mono-substituted $C_{1-6}$ aliphatic group; a saturated or unsaturated, optionally at least mono-substituted, optionally at least one heteroatom as ring member containing $C_{3-8}$ cycloaliphatic group, which may be condensed with a mono- or polycyclic ring-system; or an optionally at least mono-substituted, 5- or 6-membered aryl or heteroaryl group, which may be condensed with a mono- or polycyclic ring system and/or bonded via a methylene ($-CH_2-$) or ethylene ($-CH_2-CH_2$)-group, preferably $R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups.

**7.** Active substance combination according to one or more of claims 1-6, **characterized in that** $R^7$ and $R^8$, identical or different, represent a hydrogen atom; an unbranched or branched, saturated or unsaturated, optionally at least mono-substituted $C_{1-6}$ aliphatic radical; a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system; or an optionally at least mono-substituted, 5- or 6 membered aryl or heteroaryl group, which may be condensed with an optionally at least mono-substituted mono- or polycyclic ring system and/or bonded via a methylene ($-CH_2-$) or ethylene ($-CH_2-CH_2$)-group, preferably $R^7$ and $R^8$, identical or different, represent a hydrogen atom or a $C_{1-6}$ alkyl radical.

**8.** Active substance combination according to one or more of claims 1-7,
**characterized in that**
$R^1$ represents a phenyl group, which is optionally substituted with 1, 2, 3, 4 or 5 substituents independently selected from the group consisting of methyl, ethyl, F, Cl, Br and $CF_3$,

$R^2$ represents a phenyl group, which is optionally substituted with 1, 2, 3, 4 or 5 substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more of $C_{1-6}$-alkyl groups, or $R^3$ represents an -$NR^4R^5$-moiety,
$R^4$ represents a hydrogen atom or a linear or branched $C_{1-6}$-alkyl group,
$R^5$ represents a linear or branched $C_{1-6}$ alkyl group; an -$SO_2$-$R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different.

**9.** Active substance combination according to one or more of claims 1-8,
**characterized in that**
$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group, a piperidinyl group, a piperazinyl group, a homo-piperazinyl group, a morpholinyl group, or an -$NR^4R^5$-moiety,
$R^4$ represents a hydrogen atom or a linear or branched $C_{1-6}$-alkyl group,
$R^5$ represents a linear or branched $C_{1-6}$ alkyl group; an -$SO_2$-$R^6$-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different.

**10.** Active substance combination according to one or more of claims 1-9, **characterized in that** the pyrazoline compound of general formula I is selected from the group consisting of
N-piperidinyl-5-(4-chloro-phenyl)-1-(2,4-dichlorophenyl)-4,5-dihydro-1 H-pyrazol-3-carboxamide,
5-(4-Chloro-phenyl)-1-(2,4-dichloro-phenyl)-4,5-dihydro-1 H-pyrazole-3-carboxylic acid-[1,2,4]-triazole-4-yl-amide,
5-(4-Chloro-phenyl)-1-(2,4-dichloro-phenyl)-4,5-dihydro-1 H-pyrazole-3-carboxylic acid-(4-methyl-piperazin-1-yl)-amide,
5-(4-Chloro-phenyl)-1-(2,4-dichloro-phenyl)-4,5-dihydro-1 H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone and
N-[5-(4-Chloro-phenyl)-1-(2,4-dichlorophenyl)-4,5-dihydro-1H-pyrazole-3-carbonyl]-4-methylphenylsulfonamide,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

**11.** Active substance combination according to one or more of claims 1-10, **characterized in that** the pyrazoline compound of general formula I is one of the following compounds

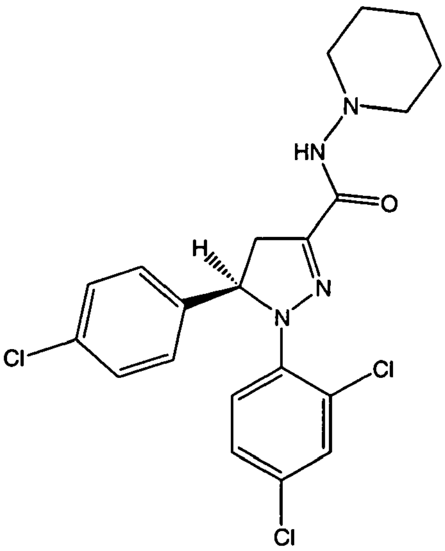

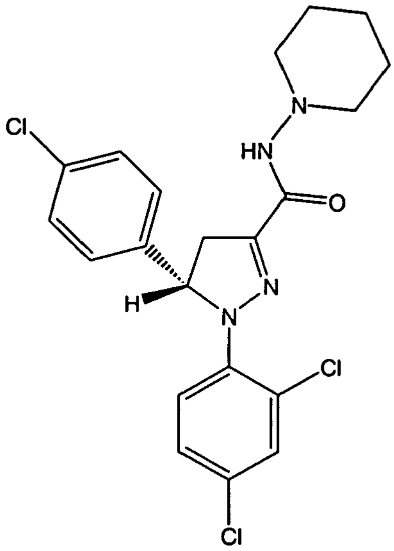

in each case optionally in the form of a corresponding N-oxide, a corresponding salt or a corresponding solvate thereof.

**12.** Active substance combination according to one or more of claims 1-11, **characterized in that** at least one of $R^{2'}$, $R^{3'}$ or $R^{4'}$ represents hydrogen, while at least one of $R^{2'}$, $R^{3'}$ or $R^{4'}$ is different from hydrogen.

**13.** Active substance combination according to one or more of claims 1-12, **characterized in that** $R^{7'}$ represents hydrogen.

**14.** Active substance combination according to one or more of claims 1-13, **characterized in that** $R^{2'}$, $R^{3'}$ and $R^{4'}$ independently of each other represent hydrogen, a linear or branched $C_{1-6}$-alkyl group, a halogen atom, or $CF_3$, preferably $R^{2'}$, $R^{3'}$ and $R^{4'}$ independently of each other represent hydrogen, methyl, ethyl, F, Cl, Br and $CF_3$.

**15.** Active substance combination according to one or more of claims 1-14, **characterized in that** $R^{5'}$ and $R^{6'}$ independently of each other represent a linear or branched $C_{1-6}$-alkyl group, a halogen atom, or $CF_3$, preferably $R^{5'}$ and $R^{6'}$ independently of each other represent methyl, ethyl, F, Cl, Br and $CF_3$.

**16.** Active substance combination according to one or more of claims 1-15, **characterized in that** $R^{2'}$ represents a chlorine atom in the 4-position of the phenyl ring, while $R^{3'}$ and $R^{4'}$ represent hydrogen.

**17.** Active substance combination according to one or more of claims 1-16, **characterized in that** $R^{5'}$ and $R^{6'}$ each represent a chlorine atoms in the 2- and 4-position of the phenyl ring, while $R^{7'}$ represents hydrogen.

**18.** Active substance combination according to one or more of claims 1-17, **characterized in that** $R^{1'}$ represents hydrogen, methyl or ethyl, preferably hydrogen.

**19.** Active substance combination according to one or more of claims 1-18, **characterized in that** the compounds of general formula I' are represented by the following structure II:

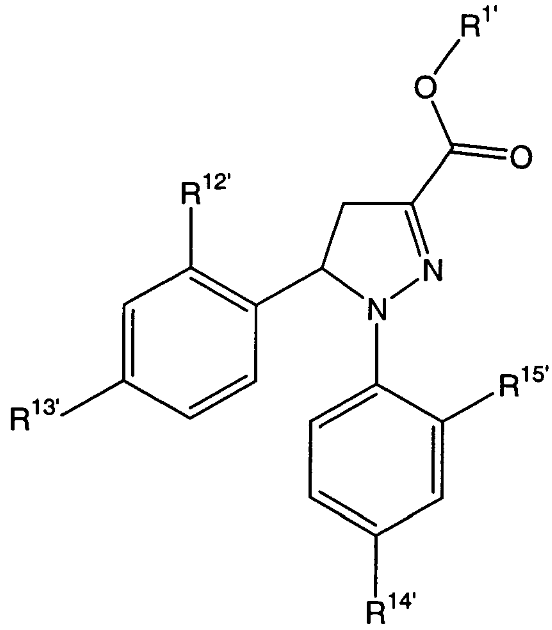

II

wherein
$R^{1'}$ represents hydrogen or a linear or branched $C_{1-4}$-alkyl group,
$R^{12'}$ or $R^{13'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,
$R^{14'}$ or $R^{15'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

**20.** Active substance combination according to claim 19, **characterized in that** $R^{12'}$ and $R^{13'}$ independently of each other represent hydrogen, a linear or branched $C_{1-6}$-alkyl group, a halogen atom, or $CF_3$, preferably $R^{12'}$ and $R^{13'}$ independently of each other represent hydrogen, methyl, ethyl, F, Cl, Br and $CF_3$.

**21.** Active substance combination according to claim 19 or 20, **characterized in that** $R^{14'}$ and $R^{15'}$ independently of each other represent a linear or branched $C_{1-6}$-alkyl group, a halogen atom, or $CF_3$, preferably $R^{14'}$ and $R^{15'}$ independently of each other represent methyl, ethyl, F, Cl, Br and $CF_3$.

**22.** Active substance combination according to one or more of claims 19-21, **characterized in that** $R^{13'}$ represents Cl and $R^{12'}$ represents hydrogen.

**23.** Active substance combination according to one or more of claims 19-22, **characterized in that** $R^{14'}$ and $R^{15'}$ each represent Cl.

**24.** Active substance combination according to one or more of claims 19-23, **characterized in that** $R^{1'}$ represents hydrogen, methyl or ethyl, preferably hydrogen.

**25.** Active substance combination according to one or more of claims 1-24,
**characterized in that** the compound of general formula I' is
5-(4-chloro-phenyl)-1-(2,4-dichlorophenyl)-4,5-dihydro-1H-pyrazol-3-carboxylic acid,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**26.** Active substance combination according to one or more of claims 1-25, **characterized in that** the pyrazoline compound of general formula I' is one of the following compounds

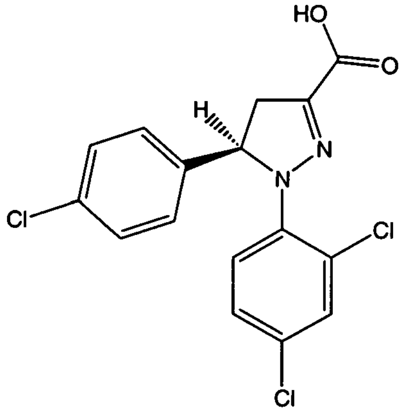 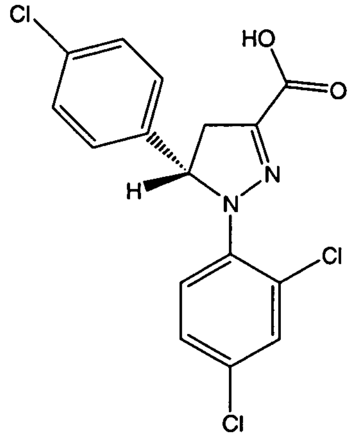

in each case optionally in the form of a corresponding N-oxide, a corresponding salt or a corresponding solvate thereof.

**27.** Active substance combination according to one or more of claims 1-26, wherein the anti-addictive agent of component (B) is an anti-alcohol-addictive agent, an anti-drug-addictive agent or an anti-nicotine-addictive agent.

**28.** Active substance combination according to claim 27, wherein the anti-alcohol-addictive agent is selected from the group consisting of Melperone, Nitrefazole, Disulfiram, Diazepam, Carbamazepine, Clobazam, Tiapride, Naltrexone, Vigabatrin, Acamprosate, Ondansetron, Nalmefene, Aripiprazole, Sodium oxybate, Rimonabant, Tryptosine (Triptosine), Ecopipam, Body protection compound-15, Ritanserin, Neramexane, Agmatine, Naltriben, Costunolide, Dehydrocostus lactone and Epipregnanolone, optionally in the form of a corresponding salt or a corresponding solvate thereof.

**29.** Active substance combination according to claim 28, wherein the anti-alcohol-addictive agent is selected from the group consisting of Melperone, Nitrefazole, Disulfiram, Diazepam, Carbamazepine, Clobazam, Tiapride, Naltrexone, Vigabatrin, Acamprosate, Ondansetron, Nalmefene, Aripiprazole, Sodium oxybate and Rimonabant, optionally in the form of a corresponding salt or a corresponding solvate thereof.

**30.** Active substance combination according to claim 27, wherein the anti-drug-addictive agent is selected from the group consisting of Disulfiram, Buprenorphine, Selegiline, Vigabatrin, Cabergoline, Nalmefene, Methadone, Naltrexone, Lofexidine, Amineptine, Flumazenil, Flumazepil, L-Deprenyl, Vanoxerine, Ecopipam, Adrogolide, Cocaine-BSA conjugate and Cocaine conjugate vaccine, optionally in the form of a corresponding salt or a corresponding solvate thereof.

**31.** Active substance combination according to claim 30, wherein the anti-drug-addictive agent is selected from the group consisting of Disulfiram, Buprenorphine, Selegiline, Vigabatrin, Cabergoline, Nalmefene, Methadone, Naltrexone, Lofexidine, Amineptine, Flumazenil and Flumazepil, optionally in the form of a corresponding salt or a corresponding solvate thereof.

**32.** Active substance combination according to claim 27, wherein the anti-nicotine-addictive agent is selected from the group consisting of Mecamylamine, Naltrexone, Anabasine, Amfebutamone; Bupropion, Topiramate, Rimonabant, Nicotine conjugate vaccine, Befloxatone, Physostigmine/Scopolamine, Cotinine Qb-Nicotine, Nicotine-Qbeta, Lazabemide, (L)-Lobeline and Varenicline, optionally in the form of a corresponding salt or a corresponding solvate thereof.

**33.** Active substance combination according to claim 32, wherein the anti-nicotine-addictive agent is selected from the group consisting of Mecamylamine, Naltrexone, Anabasine, Amfebutamone; Bupropion, Topiramate and Rimonabant, optionally in the form of a corresponding salt or a corresponding solvate thereof.

**34.** Active substance combination according to one or more of claims 1-28 comprising

(A) at least one substituted pyrazoline compound of general formula I

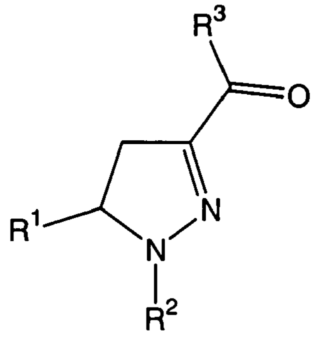

I

wherein
$R^1$ represents a phenyl group, which is optionally substituted with 1, 2, 3, 4 or 5 substituents independently selected from the group consisting of methyl, ethyl, F, Cl, Br and $CF_3$,
$R^2$ represents a phenyl group, which is optionally substituted with 1, 2, 3, 4 or 5 substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more of $C_{1-6}$-alkyl groups, or $R^3$ represents an $-NR^4R^5$-moiety,
$R^4$ represents a hydrogen atom or a linear or branched $C_{1-6}$-alkyl group,
$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different.
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and/or
at least one substituted pyrazoline compound of general formula I' represented by the following structure II:

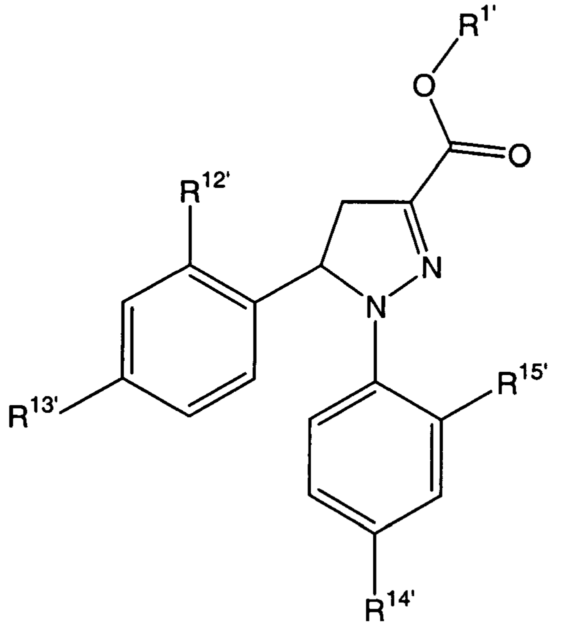

II

wherein
$R^{1'}$ represents hydrogen or a linear or branched $C_{1-4}$-alkyl group,
$R^{12'}$ or $R^{13'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,
$R^{14'}$ or $R^{15'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
and
(B) is an anti-alcohol-addictive agent, an anti-drug-addictive agent or an anti-nicotine-addictive agent.

**35.** Active substance combination according to one or more of claims 1-29 comprising

(A) one or more substituted pyrazoline compounds of general formula I selected from the group consisting of
N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1 H-pyrazole-3-yl]-piperidine-1-yl-methanone and
N-[5-(4-Chloro-phenyl)-1-(2,4-dichlorophenyl)-4,5-dihydro-1H-pyrazole-3-carbonyl]-4-methylphenylsulfonamide,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
and/or
5-(4-chloro-phenyl)-1-(2,4-dichlorophenyl)-4,5-dihydro-1 H-pyrazol-3-carboxylic acid
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof,

and
(B) is an anti-alcohol-addictive agent, an anti-drug-addictive agent or an anti-nicotine-addictive agent.

**36.** Active substance combination according to claim 34 or 35, wherein the anti-alcohol-addictive agent is selected from the group consisting of Melperone, Nitrefazole, Disulfiram, Diazepam, Carbamazepine, Clobazam, Tiapride, Naltrexone, Vigabatrin, Acamprosate, Ondansetron, Nalmefene, Aripiprazole, Sodium oxybate and Rimonabant, optionally in the form of a corresponding salt or a corresponding solvate thereof.

**37.** Active substance combination according to claim 34 or 35, wherein the anti-drug-addictive agent is selected from the group consisting of Disulfiram, Buprenorphine, Selegiline, Vigabatrin, Cabergoline, Nalmefene, Methadone, Naltrexone, Lofexidine, Amineptine, Flumazenil and Flumazepil, optionally in the form of a corresponding salt or a corresponding solvate thereof.

**38.** Active substance combination according to claim 34 or 35, wherein the anti-nicotine-addictive agent is selected from the group consisting of Mecamylamine, Naltrexone, Anabasine, Amfebutamone; Bupropion, Topiramate and Rimonabant, optionally in the form of a corresponding salt or a corresponding solvate thereof.

**39.** Active substance combination according to one or more of claims 1-38, **characterized in that** the molar ratio of component (A) to component (B) is in the range of 1:10 to 10:1, preferably 1:5 to 5:1.

**40.** Medicament comprising an active substance combination according to one or more of claims 1 to 39 and optionally at least one further active substance and/or optionally at least one auxiliary substance.

**41.** Medicament according to claim 40 for the modulation of cannabinoid-receptors, preferably cannabinoid 1 ($CB_1$) receptors; for the modulation, preferably inhibition, of an aldehyde dehydrogenase, for the modulation, preferably as agonists, of GABA(A) BZ Site Receptors, for the modulation, preferably blocking, of sodium channels, for the modulation, preferably as antagonists, of a μ-Opioid receptors, for the modulation, preferably inhibition, of a GABA aminotransferase, for the modulation, preferably inhibition, of metabotropic glutamate receptors, for the modulation, preferably as antagonists, of 5-$HT_3$ receptors, for the modulation, preferably as antagonists, of a κ-opioid-receptors, for the modulation, preferably as partial agonists, of 5-$HT_{1A}$ receptors, for the modulation, preferably as partial agonists, of 5-$HT_{2A}$ receptors, for the modulation, preferably as partial agonists, of Dopamine $D_2$ receptors, for the modulation, preferably as antagonists, of δ-Opioid receptors, for the modulation, preferably inhibiton, of MAO-B inhibitors, for the modulation, preferably inhibiton, of Prolactin secretion, for the modulation, preferably as Agonists or Antagonists, of Nicotine receptors, for the modulation, preferably inhibiton, of Dopamine Reuptake-inhibition, and/or for the modulation, preferably inhibiton, of Carbonic Anhydrase Type II.

**42.** Medicament according to claim 32 or 33 for the prophylaxis and/or treatment of abuse of and/or dependency on a substance with addictive potential.

**43.** Medicament according to claim 42, wherein the substance is alcohol, a drug or nicotine.

**44.** Medicament according to claim 43, wherein the drug is a hallucinogen, a depressant, a narcotic subtance, a steroid and/or a stimulant.

**45.** Use of at least one active substance combination according to one or more of claims 1 to 39 and optionally at least one further active substance and/or optionally at least one auxiliary substance for the manufacture of a medicament for the modulation of cannabinoid-receptors, preferably cannabinoid 1 ($CB_1$) receptors; for the modulation, preferably inhibition, of an aldehyde dehydrogenase, for the modulation, preferably as agonists, of GABA(A) BZ Site Receptors, for the modulation, preferably blocking, of sodium channels, for the modulation, preferably as antagonists, of a μ-Opioid receptors, for the modulation, preferably inhibition, of a GABA aminotransferase, for the modulation, preferably inhibition, of metabotropic glutamate receptors, for the modulation, preferably as antagonists, of 5-$HT_3$ receptors, for the modulation, preferably as antagonists, of a κ-opioid-receptors, for the modulation, preferably as partial agonists, of 5-$HT_{1A}$ receptors, for the modulation, preferably as partial agonists, of 5-$HT_{2A}$ receptors, for the modulation, preferably as partial agonists, of Dopamine $D_2$ receptors, for the modulation, preferably as antagonists, of δ-Opioid receptors, for the modulation, preferably inhibiton, of MAO-B inhibitors, for the modulation, preferably inhibiton, of Prolactin secretion, for the modulation, preferably as Agonists or Antagonists, of Nicotine receptors, for the modulation, preferably inhibiton, of Dopamine Reuptake-inhibition, and/or for the modulation, preferably inhibiton, of Carbonic Anhydrase Type II.

**46.** Use of at least one active substance combination according to one or more of claims 1 to 39 and optionally at least one further active substance and/or optionally at least one auxiliary substance for the manufacture of a medicament for the prophylaxis and/or treatment of abuse of and/or dependency on a substance with addictive potential.

**47.** Use according to claim 46, wherein the substance is alcohol, a drug or nicotine.

**48.** Use according to claim 47, wherein the drug is a hallucinogen, a depressant, a narcotic subtance, a steroid and/or a stimulant.

**49.** Pharmaceutical formulation comprising an active substance combination according to one or more of claims 1 to 39 and optionally at least one further active substance and/or optionally at least one auxiliary substance.

**50.** Pharmaceutical formulation according to claim 49, **characterized in that** it is suitable for oral or parenteral administration, preferably for oral, intravenous, intraperitoneal, intramuscular, subcutaneous, intrathekal, rectal, transdermal, transmucosal or nasal administration.

**51.** Pharmaceutical formulation for oral administration according to claim 50, **characterized in that** it is in the form of a tablet, a drageé, a powder, a capsule, drops, a gel, juice, sirup, chewing gum, solution or suspension.

**52.** Pharmaceutical formulation for oral administration according to claim 50, **characterized in that** it is in form of multiparticulates, preferably microparticles, microtablets, pellets or granules, optionally compressed into a tablet, filled into a capsule or suspended in a suitable liquid.

**53.** Pharmaceutical formulation for oral administration according to claims 50-52, **characterized in that** it comprises at least one enteric coating.

**54.** Pharmaceutical formulation according to claim 49-53 **characterized in that** it comprises component (A) and/or component (B) at least partially in a sustained-release form.

**55.** Pharmaceutical formulation according to claim 54, **characterized in that** the sustained release is achieved by at least one coating or matrix comprising at least one sustained-release material.

**56.** Pharmaceutical formulation according to claim 54, **characterized in that** the sustained-release material is based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

**57.** Pharmaceutical formulation according to claim 56, **characterized in that** the water-insoluble polymer is based on an acrylic resin, which is preferably selected from the group of poly(meth)acrylates, poly($C_{1-4}$)dialkylamino($C_{1-4}$) alkyl (meth)acrylates and/or copolymers thereof or a mixture of at least two of the afore-mentioned polymers.

**58.** Pharmaceutical formulation according to claim 56, **characterized in that** the water-insoluble polymers are cellulose derivatives, preferably alkyl cellulose, particularly preferably ethyl cellulose, or cellulose esters.

**59.** Pharmaceutical formulation according to claim 56, **characterized in that** the wax is carnauba wax, beeswax, glycerol monostearate, glycerol monobehenate, glycerol ditripalmitostearate, microcrystalline wax or a mixture of at least two of these components.

**60.** Pharmaceutical formulation according to any one of claims 56-59, **characterized in that** the polymers have been used in combination with one or more plasticizers.

**61.** Pharmaceutical formulation according to any one of claims 49-60, **characterized in that** it comprises component (A) and/or (B) in immediate-release form as well as in sustained release form.

European Patent Office

# EUROPEAN SEARCH REPORT

Application Number
EP 05 38 4009

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GB 2 359 488 A (BRITANNIA PHARMACEUTICALS LTD [GB]) 29 August 2001 (2001-08-29) * claims 1-3 * | 1-61 | INV. A61K31/415 A61K31/4152 A61P25/30 A61P25/32 A61P25/34 A61P25/36 |
| A | WO 93/12791 A (WARNER LAMBERT CO [US]) 8 July 1993 (1993-07-08) * page 12, lines 30-35 * * claims 19,21 * | 1-61 | |
| A | EP 1 384 477 A1 (ESTEVE LABOR DR [ES]) 28 January 2004 (2004-01-28) compounds 34-36 * claim 1 * | | |
| A | EP 0 429 360 A2 (ESTEVE LABOR DR [ES]) 29 May 1991 (1991-05-29) * abstract * | 1-61 | |
| E | WO 2005/077911 A (ESTEVE LABOR DR [ES]; CUBERES ALTISEN ROSA [ES]; FRIGOLA CONSTANSA JOR) 25 August 2005 (2005-08-25) * claims 1,9,32,35 * | 1-61 | TECHNICAL FIELDS SEARCHED (IPC) A61K |

The present search report has been drawn up for all claims

2

| Place of search | Date of completion of the search | Examiner |
|---|---|---|
| Munich | 12 December 2006 | Collura, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 38 4009

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2006

| Patent document cited in search report | | Publication date | Patent family member(s) | | | Publication date |
|---|---|---|---|---|---|---|
| GB 2359488 | A | 29-08-2001 | AT | 289194 | T | 15-03-2005 |
| | | | AU | 3755001 | A | 12-09-2001 |
| | | | DE | 60108947 | D1 | 24-03-2005 |
| | | | DE | 60108947 | T2 | 27-10-2005 |
| | | | EP | 1267849 | A2 | 02-01-2003 |
| | | | ES | 2236183 | T3 | 16-07-2005 |
| | | | WO | 0164201 | A2 | 07-09-2001 |
| | | | US | 2004077636 | A1 | 22-04-2004 |
| WO 9312791 | A | 08-07-1993 | EP | 0617621 | A1 | 05-10-1994 |
| | | | JP | 7507993 | T | 07-09-1995 |
| EP 1384477 | A1 | 28-01-2004 | AT | 326966 | T | 15-06-2006 |
| | | | BR | 0208805 | A | 13-07-2004 |
| | | | CA | 2442974 | A1 | 17-10-2002 |
| | | | CN | 1509171 | A | 30-06-2004 |
| | | | CN | 1698602 | A | 23-11-2005 |
| | | | DK | 1384477 | T3 | 21-08-2006 |
| | | | WO | 02080909 | A1 | 17-10-2002 |
| | | | ES | 2174757 | A1 | 01-11-2002 |
| | | | HU | 0400918 | A2 | 28-07-2004 |
| | | | IS | 6973 | A | 03-10-2003 |
| | | | JP | 2004525166 | T | 19-08-2004 |
| | | | MA | 27019 | A1 | 20-12-2004 |
| | | | MX | PA03009124 | A | 11-04-2005 |
| | | | NO | 20034470 | A | 05-12-2003 |
| | | | NZ | 529304 | A | 24-02-2006 |
| | | | PL | 365220 | A1 | 27-12-2004 |
| | | | PT | 1384477 | T | 29-09-2006 |
| | | | UA | 75402 | C2 | 15-01-2004 |
| | | | US | 2004034082 | A1 | 19-02-2004 |
| | | | ZA | 200308626 | A | 05-11-2004 |
| EP 0429360 | A2 | 29-05-1991 | AU | 628237 | B2 | 10-09-1992 |
| | | | AU | 6676790 | A | 30-05-1991 |
| | | | CA | 2030401 | A1 | 23-05-1991 |
| | | | DE | 69003924 | D1 | 18-11-1993 |
| | | | DE | 69003924 | T2 | 24-02-1994 |
| | | | DK | 429360 | T3 | 13-12-1993 |
| | | | ES | 2034909 | A6 | 01-04-1993 |
| | | | FR | 2654621 | A1 | 24-05-1991 |
| | | | HU | 56093 | A2 | 29-07-1991 |
| | | | JP | 1972937 | C | 27-09-1995 |
| | | | JP | 3176422 | A | 31-07-1991 |
| | | | JP | 6099309 | B | 07-12-1994 |
| | | | NO | 905028 | A | 23-05-1991 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 38 4009

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2006

| Patent document cited in search report | Publication date | Patent family member(s) | Publication date |
|---|---|---|---|
| EP 0429360 A2 | | PT 95949 A | 13-09-1991 |
| | | RU 2042674 C1 | 27-08-1995 |
| | | US 5162323 A | 10-11-1992 |
| | | ZA 9009256 A | 30-10-1991 |
| WO 2005077911 A | 25-08-2005 | AU 2005212836 A1 | 25-08-2005 |
| | | CA 2556400 A1 | 25-08-2005 |
| | | EP 1626963 A1 | 22-02-2006 |
| | | US 2006020010 A1 | 26-01-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Synthetic communications,* 1996, vol. 26 (11), 2229-33
- *Synlett,* 2001, 147-149
- **PASCUAL, A.** *J. Prakt Chem.,* 1999, vol. 341 (7), 695-700
- **LIN, S. et al.** *Heterocycles,* 2001, vol. 55 (2), 265-277
- **RAO, P. et al.** *J. Org. Chem.,* 2000, vol. 65 (22), 7323-7344
- **PEARSON D.E ; BUEHLER, C.A.** *Synthesis,* 1972, 533-542
- **KROGSGAARD-LARSEN et al.** Textbook of Drugdesign and Discovery. Taylor & Francis, April 2002
- **WILSON et al.** *Am. J. Cardiol.,* 1987, vol. 59 (14), 91G-94G
- Pharmaceutics: the Science of Dosage Forms. Churchill Livingstone, 2002
- Encyclopedia of Pharmaceutical Technology. Marcel Dekker, Inc, 2002
- Modern Pharmaceutics. Marcel Dekker, Inc, 2002
- The Theory and Practice of Industrial Pharmacy. Lea & Febiger, 1986
- Modified-Release Drug Delivery Technology. Marcel Dekker, Inc, 2002
- Handbook of Pharmaceutical Controlled Release Technology. Marcel Dekker, Inc, 2000
- Controlled Drug Delivery. Basic Concepts. CRC Press Inc, 1983, vol. I
- Oral Drug delivery. **TAKADA, K. ; YOSHIKAWA, H.** Encyclopedia of Controlled Drug Delivery. John Wiley & Sons, Inc, 1999, vol. 2, 728-742
- Oral drug delivery, small intestine and colon. **FIX, J.** Encylopedia of Controlled Drug Delivery. John Wiley & Sons, Inc, 1999, vol. 2, 698-728
- Pharmaceutical tablet coating. **JOHNSON, J.L.** Coatings Technology Handbook. Marcel Dekker, Inc, 2001, 863-866
- **CARSTENSEN, T.** Coating Tablets in Advanced Pharmaceutical Solids. Marcel Dekker, Inc, 2001, 455-468
- **LEOPOLD, C.S.** Coated dosage forms for colon-specific drug delivery. *Pharmaceutical Science & Technology Today,* 1999, vol. 2 (5), 197-204
- **RHODES, C.T. ; PORTER, S.C.** Coatings, in Encyclopedia of Controlled Drug Delivery. John Wiley & Sons, Inc, 1999, vol. 1, 299-311
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **A. C. HOWLETT et al.** Intemational Union of Pharmacology XXVII. *Classification of Cannabinoid Receptors, Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282